# EUROPEAN PATENT SPECIFICATION

(11) **EP 2 319 515 B1**
(45) Date of publication and mention of the grant of the patent: **07.05.2014**
(21) Application number: 09802583.6
(22) Date of filing: 30.07.2009
(51) Int. Cl.: A61K 31/53, A61K 31/505, A61K 33/02, A61K 45/06, A61K 9/10, A61K 49/00, A61K 47/10, G01N 1/00

(54) **COCCIDICIDE COMBINATION FOR VETERINARY USE**
KOMBINATION AUS KOKZIDIOSTATIKA FÜR VETERINÄRMEDIZINISCHE ANWENDUNG
COMBINAISON COCCIDICIDE À USAGE VÉTÉRINAIRE

(30) Priority: 31.07.2008 MX 2008009818
(43) Date of publication of application: 11.05.2011
(73) Proprietor: Laboratorio Avi-Mex, S.A. De C.V., México, D.F. 09810 (MX)
(72) Inventor: LOZANO-DUBERNARD, Bernardo, Distrito Federal 04660 (MX); OCAMPO-CAMBEROS, Luis, Distrito Federal 16300 (MX); SUMANO-LOPEZ, Héctor, Salvador, Distrito Federal 04620 (MX); SOTO-PRIANTE, Ernesto, Distrito Federal 01900 (MX); SARFATI-MIZRAHI, David, Edo . de México 52786 (MX)
(74) Representative: Wibbelmann, Jobst
(86) International application number: PCT/IB2009/006409
(87) International publication number: WO 2010/013129

(56) References cited:
- WO-A1-98/43644
- Anonymous: "45626: BAYCOX COCCIDIOCIDE SOLUTION", Bayer Animal Health , 17 February 2006 (2006-02-17), XP002659608, Retrieved from the Internet: URL:http://www.bayeranimal.com.au/PDFViewe r/PDFDownload.aspx?DocumentOID=X0mKcwwdzRX 8tg2cpeF5WQ== [retrieved on 2011-09-21]
- Lorraine Shelton: "Use of Baycox liquid or Marquis paste to control coccidiosis incatteries", , 6 January 2009 (2009-01-06), XP002659609, Retrieved from the Internet: URL:http://www.fanciers.com/index.php?view =items&cid=17%3AInternal+Parasites&id=81%3 Atreating-coccidia&format=pdf&option=com_q uickfaq&Itemid=165 [retrieved on 2011-09-21]
- HARDER, A. ET AL.: 'Possible mode of action of toltrazuril: studies on two Eimeria species and mammalian and Ascaris suum enzymes' PARASITOL. RES. vol. 76, 1989, pages 8 - 12, XP008142774
- VOYVODA, H. ET AL.: 'Clinical Hepatozoon canis infection in a dog in Turkey' JOURNAL OF SMALL ANIMAL PRACTICE vol. 45, no. 12, 2004, pages 613 - 617, XP008142757
- ACHIM HARDER ET AL: "Possible mode of action of toltrazuril: studies on twoEimeria species and mammalian and Ascaris suum enzymes", PARASITOLOGY RESEARCH, SPRINGER VERLAG, BERLIN, DE, vol. 76, no. 1, 1 January 1989 (1989-01-01), pages 8-12, XP008142774, ISSN: 0932-0113, DOI: 10.1007/BF00931064

## Description

### TECHNICAL FIELD

The present invention is related to the techniques used for the obtainment and preparation of veterinary and animal husbandry compositions, medicines and drugs, and more particularly, it is related to a coccidicide combination based on the compound 1-methyl-3-[3-methyl-4-[4-(trifluoromethyl)tio)phenoxil]phenyl]-1,3,5-triazine(1H,3H,5H)-2,4,6-trione, better known as toltrazuril, and the compound 5-[(3,4,5-trimethoxyphenyl)methyl]pirimidyn-2,4-diamine, known as trimethoprim; both compounds in combination showing a synergistic effect in accelerating the recovery from coccidiosis for birds and other animals. In addition, when added in the drinking water for its use, the combination of the present invention has an improved stability.

### BACKGROUND OF THE INVENTION

Coccidiosis is a disease affecting the digestive tract of diverse birds, and mammals such as pigs, bovine, cats and dogs. Said avian disease is caused by the *Eimeria sp* genre protozoa, from which 9 species have been identified, being of the higher incidence in Mexico's poultry, mainly in chicken, four of them, said four species being *Eimera acervulina, Eimera maxima, Eimera necatrix* and *Eimera tenella,* which year after year cause severe damages to the poultry industry.

Although extensive research on the control and treatment of the coccidiosis has been done, this parasitic disease is still one of the most expensive in the poultry industry. Coccidiosis is transmitted by direct or indirect contact with birds infected feces. Particularly, when the bird ingests such protozoa, also named coccidia, the disease onsets invading the bird's intestinal mucous, causing tissue injuring as its reproduces. About a week after the infection, the protozoa produces descendants named oocytes, which when expelled with the feces cannot infect another bird until reaching a maturity process (sporulation), which occurs in a period of time from one to three days. If ingested by another bird after the sporulation, the protozoa are capable of causing infection.

The severity of the infestation will depend on the strain pathogenicity and the number of infective coccidias ingested by the bird, e.g., the infestation may be soft enough to be imperceptive, while a very high amount of coccidias may cause severe injuries ending in death. The coccidias are easily transmitted from a coop to another by means of contaminated shoes or clothing, free birds, equipment, food sacs, insects and rodents.

Although coccidiosis generally occurs in growing and young adults chicken, it may also occur in other stages. A coccidiosis outbreak can be detected on wake and pale birds tending to nestle, which consume less food and water, with diarrhea and dehydration signs. Laying birds with coccidiosis show a low laying.

The injuries produced by the infestation depend on the coccidian species, the severity and the stage of the disease. Cecal coccidiosis may cause cecal swelling, which fills with fresh blood. In a later stage, the blind becomes full of a caseous-like material, and variably bloody stained. Intestinal coccidiosis injuries range from a very soft enteritis up to a necrotic or hemorrhagic type one.

Now, from a commercial perspective, since a bird having had coccidiosis lose its skin pigmentation, which turns to a yellowish color due to a lack of assimilation of the pigments included in the food when the bird is ill, it loses value, then, it is crucial to attack the coccidiosis in an effective manner.

Regarding the prevention and control methods, it is almost impossible to prevent coccidiosis with a good hygiene only. Therefore, anti-coccidian compositions have been developed in the prior art to attack these kinds of parasites, the anti-coccidian products being of chemical or biological nature. According to the administration route, the chemical anti-coccidian are generally classified as coccidicides typically mixed or added to the birds' food, and anti-coccidian typically added to the birds' drinking water.

Nicarbazin, sulfonamides (sulfas), diclazuril, ethopabate, clopidol, decoquinate, robenidine, and 3-nitro-4-hydroxy-pheliarsonic acid, as well as ionophores compounds such as maduramicine, salinomycin, lasalocid and monensine are among the chemical products added to the food, generally being of a preventive nature.

Sulfonamides, amprolium and toltrazuril, among others, are coccidicides chemicals of a therapeutic nature. In addition, vaccines have also been developed, but these are of a minor general use due to the costs, administration difficulties and variable effectiveness.

From the above-mentioned compounds, toltrazuril is without any doubts one of the most used compounds for the treatment of coccidiosis in birds. In this regard, North American patents 4,631,218; 5,141,938 and 5,256,631; as well as the European patent EP 1 246 624 disclose anti-coccidian products including toltrazuril. In the commerce, the toltrazuril for avian use may be purchased, e.g., under the trademark Baycox® 2.5% from Bayer. This toltrazuril-based formulation has shown an acceptable performance, however, it is always desirable to have more effective compositions causing a faster recovery of birds suffering from the disease, and fewer side effects, i.e., the aviculturists always desire more effective formulations.

On the other hand, however, the use of toltrazuril-based formulations has shown administration issues, such like a rapid precipitation in water of the compositions when solubilized, causing a lack of assimilation by the birds.

In other words, traditionally, the administration of the toltrazuril has been carried out by pouring and diluting it in the containers supplying water to the birds drinking trough, however, the drinking troughs are formed by small diameter piping and filters which may be plugged by the toltrazuril when precipitates, therefore, the birds do not get the appropriate medicament doses.

Further, drinking water having a high carbonate and salts contents, i.e., hard-water, is an additional problem found in many poultry farms, since toltrazuril loses effectiveness when mixed with this kind of water. In fact, it is calculated that the toltrazuril loses about 60% of its effectiveness when mixed with hard-water, said water being very common in the central and north part of Mexico, as well as in many parts worldwide. The obvious consequences are that birds do not receive the appropriate doses of active toltrazuril, and that aviculturists require investing extra money to buy additional medicament to provide the birds with the recommended toltrazuril doses.

Then, in order for the commercially available toltrazuril to keep it effectiveness, it requires to be mixed with water having low salts content, and this is hardly complied in Mexico and in other countries.

Summarizing, coccidicide compositions are required having an enhanced effectiveness for a faster recovery of the infected animals with fewer side effects, and coccidicide compositions which do not lose effectiveness when being mixed with any kind of water.

### BRIEF DESCRIPTION OF THE INVENTION

The combination of 1-methyl-3-[3-methyl-4-[4-(trifluoromethyl)tio)phenoxil]phenyl]-1,3,5-triazine(1 H,3H,5H)-2,4,6-trione, better known as toltrazuril; and the compound 5-[(3,4,5-trimethoxyphenyl)methyl]pirimidyn-2,4-diamine, known as trimethoprim, has been found as having a synergistic effect for the treatment of coccidiosis. This combination achieved an enhanced therapeutic effect for coccidiosis during the experimental trials, explained below, with respect to each compound alone, particularly, the birds shown faster recovery signs with fewer side effects with respect to the prior art compositions.

In a preferred embodiment, the best weight ratio as found between the components of the combination of the present invention is in the range from 1:1 to 1:5 (trimethoprim/toltrazuril).

In the invention a coccidicide composition is provided, comprising toltrazuril, trimethoprim and a pharmaceutically acceptable vehicle, such composition is formulated for oral administration. Preferably, for oral administration a solution or suspension is used, the solution being formed by mixing with water. Direct administration to the oral cavity of each animal is a further administration route of the composition.

Further disclosed is the use of a therapeutically effective amount of a combination formed by toltrazuril and trimethoprim, to make a medicament useful in the treatment of coccidiosis in birds and mammals. Further a method for the treatment of coccidiosis in birds and mammals is provided, by using a therapeutically effective amount of the combination formed by toltrazuril and trimethoprim.

### BRIEF DESCRIPTION OF THE DRAWINGS

The novel aspects considered characteristics of the present invention are particularly set forth in the appended claims, however, the combination of the present invention, the veterinary composition and the use as anti-coccidian thereof will be better understood from the following detailed description of certain preferred embodiments, when read related to the appended drawings, wherein:

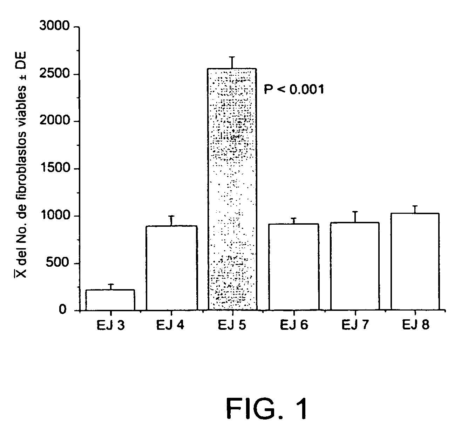

Figure 1 is a graph showing the results obtained in comparative *in vivo* trials using an embodiment of the combination of the present invention and the prior art coccidicide compositions, the trials were carried out in chicken fibroblasts.

### DETAILED DESCRIPTION OF THE PREFERRED EMBODIMENTS OF THE INVENTION

In the present invention, a synergistic effect from a combination of the compound 1-methyl-3-[3-methyl-4-[4-(trifluoromethyl)tio)phenoxil]phenyl]-1,3,5-triazine(1 H,3H,5H)-2,4,6-trione, hereinafter referred as toltrazuril; and the compound 5-[(3,4,5-trimethoxyphenyl)methyl]pirimidyn-2,4-diamine, hereinafter referred as trimethoprim, was found for the treatment of coccidiosis, which in particular, helps to a faster recovery of the ill birds and with fewer side effects than each compound alone. In a preferred embodiment of the present invention, a weight ratio of trimethoprim to toltrazuril from 1:1 to 5:1 (trimethoprim/toltrazuril) is preferred.

The first component of the combination, toltrazuril, is a triazinetrione derivative which use has been well known to prevent and treat coccidiosis in chicken, turkeys and pigs by administration to the animals' drinking water or directly in the oral cavity.

The toltrazuril has the following chemical structure (I).

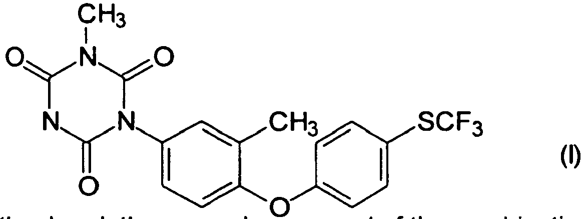

On the other hand, the second compound of the combination of the present invention, trimethoprim, has the following chemical structure (II):

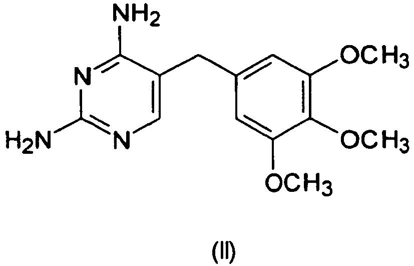

The trimethoprim is a bacteriostatic antibacterial mainly prophylactically used in the urinary tract infections. During the development of the present invention, the best weight ratio between both components was found, being from 1:1 to 5:1 (trimethoprim/toltrazuril); below said ratio the combination does not have the required therapeutic effect, and above said ratio some related to the formulation precipitation may occur, especially from trimethoprim.

A veterinary combination is formulated for the application of the combination of the present invention, employing organic and inorganic liquids and solids as pharmaceutically acceptable vehicles, selected from starch, lactose, glucose, sucrose, dextrin, cellulose, paraffin, water, alcohol, polyethylene glycol, propylene glycol, triethanolamine, Arabic gum, carbopol, poloxamers, chitosan, alpha, beta and gamma cyclodextrines, dimethyl sulfoxide, etc.

The veterinary composition of the present invention is formulated for oral administration, more preferably, the formulation of the composition in a suspension form for oral administration is preferred. Moreover, an oral administration suspension, comprises from 20 to 30 mg of toltrazuril per ml of suspension; and from 20 to 150 mg of trimethoprim per ml of suspension. Below the minimum value indicated for each component, the composition simple does not has the required effectiveness, while with higher values specially of the trimethoprim, the composition precipitates. The suspension or solution may be mixed with water in a ratio higher or equal to 1:1000 (solution/water) and it may have a basic pH, preferably, from 8.5 to 12.5.

The veterinary composition formulated for oral administration may be applied to birds from 1 day old and older, for those birds having from 10 to 18 days old, the composition may be administered during 1 or 2 consecutive days. The recommended doses are from 5 to 10 mg/kg by weight for two consecutive days.

According to the present invention, the combination with synergistic effects of toltrazuril and trimethoprim is used to manufacture a veterinary medicament useful in the treatment of coccidiosis in birds and mammals, more particularly, its application in poultry selected from a group comprising of chicken, turkeys, ducks and gooses is preferred, more preferably applied to chicken.

The combination of the present invention, its use in a pharmaceutical combination and its synergic effect when applied to birds, will be better illustrated by the following specific examples, which should only be considered as illustrative and non-limitative of the invention.

### EXAMPLES 1-6

### in vitro comparative trials for effectiveness

*Rho0 line, cellular line CS-3* chicken fibroblasts, were used to evaluate the *in vitro* efficacy of the combination of the present invention, [Gurnett et al., 1995 A.M. Gumett, P.M. Dulski, S.J. Darkin-Rattray, M.J. Carrington and D.M. Schmartz, Selective labeling of intracellular parasite proteins by using ricin, Proceedings of the National Academy of Sciences of the United States of America 92: 2388-2392*],* the fibroblast were maintained at 41°C at the presence of 5% CO₂ in Ham's media F-12-Glutamax, supplemented with 10% heat inactivated bovine fetal serum, 100 Ul/ml penicillin, 100 mg/ml streptomycine, 50 mg/ml uridine and 1 mM sodium pyruvate.

*Eimerias.-* Stain PAPt37 of *Emeria tenella* oocytes non-sporulated cultures were maintained, obtained from the "Departamento de Parasitologia de la Facultad de Medicina Veterinaria y Zootécnica" from the Universidad Nacional Autónoma de México (Parasitology Department of the Medicine Veterinary and Husbandry Faculty of the Mexican National University). Sporocytes by sporulated oocyte encystment were obtained as described in Dulsky and Turner, 1988 P. Dulski and M. Turner. The Purification of sporocysts and sporozoites from Eimeria Tenella oocysts using Percoli density gradients, Avian Diseases 32 (1988), pp. 235-239. Merozoites were purified from the cecal content 108 h after the inoculation of experimental chickens with a charge of 10⁶ oocytes as suggested by Binger et al., 1993 M.H. Binger, D. Hug, G. Weber, E. Schildknecht, M. Humbelin and L. Pasamontes, Cloning characterization of a surface antigen of Eimeria Tenella merozoites and expression using a recombinant vaccinia virus, Molecular and Biochemical Parasitology 61 (1993), pp 179-187*.*

The anti-coccidian efficacy of the following mixtures was compared at the specified concentrations.

### Example 1. Maduramicine (10 mg/ml) (as witness of the trial efficacy)

### Example 2. Toltrazuril (5 mg/ml)

### Example 3. Trimethoprim + toltrazuril (5 mg/ml + 1 mg/ml)

### Example 4. Maduramicine + toltrazuril (5 mg/ml + 5 mg/ml)

### Example 5. Toltrazuril + diminazene diaceturate (5 mg/ml + 10 mg/ml)

### Example 6. Toltrazuril + pyrimethamine (5 mg/ml + 2 mg/ml)

*Bioassays.-* The infection studies were started with the *E. tenella* culture, by forming fibroblasts plates in tissue culture vials, with an initial inoculum of 100 ml of a 8 x 10⁴ cells suspension, the cell were growth for 48 hours at 41°C. Further, they were infected with *E. tenella* sporocytes in an approximate ratio of 3 sporocytes per cell. The following sub-groups for each drug or mix of drugs were managed by triplicate for each example 1 to 6.
A. Fibroblasts + sporocytes only
B. Fibroblasts + sporocytes + maduramicine (example 1)
C. Fibroblasts + sporocytes + trial mix (examples 2 to 6)

At 24 hours of incubation, the culture media was carefully removed, the cells were dried with liquid nitrogen injecting the vials, and fixed with neutral 10% formalin. The vials bottoms were trimmed and the cultures stained with hematoxylin-eosine. The cultures were analyzed by a microscope at 40 X and quantified, the cells had an affected or infected structure in 9 fields, distinguishable from the non-altered cells. A U Mann-Whitney non-parametric statistical analysis was made.

*Results.-* The obtained data are shown In Table 1, as X ± 1 SD (Standard Deviation). The statistical analysis revealed that the group of Example 3, toltrazuril-trimethoprim, is statistically better in protecting the fibroblasts from destruction by the *Eimeria tenella* sporocytes (P < 0.01).

**TABLE 1**

| **Replication** | **Example 1 (±SD)** | **Example 2 (±SD)** | **Example 3 (±SD)** | **Example 4 (±SD)** | **Example 5 (±SD)** | **Example 6 (±SD)** |
|---|---|---|---|---|---|---|
| A | 268 (29) | 999 (12) | 2569 (26) | 879 (28) | 789 (25) | 945 (23) |
| B | 156 (35) | 899 (8) | 2687 (28) | 985 (26) | 1002 (23) | 1021 (28) |
| C | 239 (21) | 789 (6) | 2459 (32) | 875 (34) | 985 (30) | 1106 (20) |
| Means mean | 221 (58) | 895 (105) | 2571 (114) | 913 (62) | 925 (118) | 1024 (80) |

The above data are shown in the form of a frequency histogram in Figure 1.

According to the above trials, the established system is capable of detecting *in vitro* activity differences from the different active ingredients. The toltrazuril-trimethoprim combination (example 3) is synergistic to destroy *Eimeria sp* and to improve the viability of the host cells or "target", which would translate in a less birds' feelings on the effects of the disease, and for they to have a faster recovery.

### EXAMPLE 7

### In vivo trials comparative example

Particularly, in order to compare the effectiveness of the present invention with respect to the commercial drug Baycox® 2.5% (Bayer de México), a 1 ml/L dose of a suspension prepared according to Table 2 and a dose of the commercial product Baycox® were used at the 5^{th} and 6^{th} day post-inoculation, in *E*. *tenella* infected experimental chicken.

**TABLE 2**

| **Compositions employed in Example 3** | |
|---|---|
| **Components** | **Example 3** |
| Toltrazuril | 0.5% |
| Triethanolamine | 30% |
| Polyethylene glycol | 57.5% |
| trimethoprim | 2.5% |
| Ethanol | 7.5% |
| pH | 11.2 |

More particularly, 180 (male) commercial broilers were used of 1 day old and only of one gender, broilers were grown together until the 3^{rd} week. From this date, 3 groups were formed based on weight uniformity.

For the 1-28 growing stage, commercial broiler food with an anti-coccidian was used. As from the group formation date at day 28 and up to the birds sacrifice on day 35, starter broiler food was administered without anti-coccidian. The feeding was at all times *ad limitum,* so was water.

Broiler were grown in 40 cm high, 50 cm wide and 1 m long wire cages, for 1 to 28 days. The same equipment was used when the trial groups were formed at days 21 to 28.

Each group had 30 broilers, having a total of two replications. Broilers were weighted in order to having groups as uniform as possible. The three formed groups are shown in Table 3.

**TABLE 3**

| **Formed Groups** | | | |
|---|---|---|---|
| **Group** | **Treatment** | **Food** | **Replications/chicken** |
| 1 | Treated with the suspension of the present invention-2 days, from the 5^{th} post-inoculation. Dose 1 ml/L | Without anti-coccidian | 2 x 30 each |
| 2 | Treated with Baycox® --2 days, from the 5^{th} post-inoculation. Dose 1 ml/L | Without anti-coccidian | 2 x 30 each |
| 3 | Inoculated Non-treated | Without anti-coccidian | 2 x 30 each |

An *E. tenella* field strain-based fresh inoculum was used, administrating a dose of 100 000 sporulated oocysts by bird thereof. The titrated sporulated inoculum was administered directly in the birds' bill by deposition in the proventricle with the aid of a stiff probe having a graduated syringe on the 28^{th} days old.

On 35 day old, all broilers were weighted and sacrificed. Necropsy was carried out for each broiler, and the blind injuries were scored according to the Johnson and Reid scale. With the obtained values at the injuries scoring, variance analysis were made (Kruskal-Wallis) my means of the SAS statistical program:
The measured parameters were as follows:
   - Weight average per group at the time of inoculation (28 days)
   - Weight average per group at the time of sacrifice (35 days)
   - Accumulated food ingestion at the exposure stage (per treatment)
   - Conversion index per treatment
   - Injuries scoring of all broilers and average per treatment
   - Injuries average per treatment
   - Signs and mortality

The obtained results are shown in Tables 4 to 7.

**TABLE 4**

| **Weight gain** | | | |
|---|---|---|---|
| **Treatments** | **Starting average weight/bird** | **Final average weight/bird** | **Average gain/bird** |
| Treated with the suspension of the present invention -2 days, from de 5th post-inoculation. Dose 1 ml/L | 520 | 695 | 175 |
| Treated with Baycox® --2 days, from the 5^{th} post-inoculation. Dose 1ml/L | 525 | 694 | 169 |
| INOCULATED NON-TREATED | 525 | 640 | 115 |

**TABLE 5**

| **Feeding conversion index** | | | |
|---|---|---|---|
| **Treatment** | **Average ingestion/bird** | **Average weight/ bird** | **Average I.C.** |
| Treated with the suspension | 376 | 147 | 2.55 |
| Baycox® | 385 | 125 | 3.08 |
| Witness | 371 | 124 | 2.99 |

**TABLE 6**

| **Average injuries level per group with relation to the control** | | |
|---|---|---|
| **Group** | | **Total** |
| Treated with the suspension | 8/20 1+=8; 10/20 3+=30; 2/20 4+=8 | Level 46 - 19 with reference to the control |
| Baycox® | 3/20 1+=3; 14/20 3+=42; 3/20 4+=12 | Level 57 -8 with reference to the control |
| Witness or control | 15/20 3+=45; 5/20 4+=20 | Level 65 |

**TABLE 7**

| **Mortality and clinical signs (5^{th} and 6^{th} day post-inoculation)** | | |
|---|---|---|
| | **Bloody feces** | **Mortality** |
| Treated with the suspensión | + | 3/20 (15%) |
| Baycox® | ++ | 3/20 (15%) |
| Witness | +++ | 5/20 (25%) |

According to the variance analysis (ANOVA) from the values found in the injury scoring, significant differences were found P< .05 between the non-treated infected group with relation to both treatments. Particularly, the weight gain and the injuries level using the composition of the present invention shows advantaging results with respect to those with the commercial treatment of Baycox® product.

The synergistic behavior of the present invention could be explained as follows: the toltrazuril and the trimethoprim are capable of stimulating the excysont death in the coccidiasis by inhibiting the respiratory chain, in particular at the cytochrome-C reductase and NADH oxidase level, as well as via the succinate oxidase. In birds, the pyrimidine synthesis is rather based in the dihydrophilic acid reductase; and, the effect of the trimethoprim and toltrazuril is 500 fold weaker with respect to coccidias, thus these two drugs prevent a possible toxicity *[*Harder A; Haberkorn A possible mode of action of toltrazuril: studies on two Eimeria species and mammalian and Ascaris suum enzymes. Parasitol Res 1989; Vol. 76 (1), pp. 8-12*].* Then, it may be perceived that a double effect on the same enzymatic groups is achieved, thus decreasing the generation of *Eimeria sp.* resistant strains.

In the clinical field, the necropsy injuries and the signs of bloody diarrhea disappear faster with the combination of the present invention, due to the two drugs double effect above-mentioned over the oxidative systems to which *Eimerias* would not generate resistance.

### EXAMPLES 8 AND 9

### Hard-water trials with a trimethoprim variable concentration

Two veterinary compositions were prepared according to the enlisted percentages in Table 8, with the purpose of analyzing the composition behavior in hard-water when varying the trimethoprim concentration.

Each veterinary composition was prepared as follows: in a container having polyethylene glycol, a predetermined amount of trimethoprim was slowly added up to the concentration indicated in Table 8 and it was mixed for 10 minutes. Subsequently, triethanolamine was added and mixed for 5 more minutes, then, a sufficient amount of toltrazuril was added to achieve the concentration indicated in Table 8, and it was mixed additionally for 5 minutes. Then ethanol was added and the container was covered, the mixture was heated with stirring at 40 °C for further 30 minutes. At the end of this operation, a white cloudy-like suspension was yielded.

**TABLE 8**

| **Compositions employed in Examples 8 and 9** | | |
|---|---|---|
| **Components** | **Example 8 5% Trimethoprim** | **Example 9 2.5% Trimethoprim** |
| Toltrazuril | 2.5% | 2.5% |
| Triethanolamine | 30% | 30% |
| Polyethylene glicol | 57.5% | 57.5% |
| Trimethoprim | 5% | 2.5% |
| Ethanol | 5% | 7.5% |
| pH | 12.5 | 11.2 |

Each one of the veterinary compositions in Examples 8 and 9 was diluted with water having different levels of hardness, as indicated in Table 9, wherein the solubility and precipitation was quantitatively determined for the compositions of Examples 8 and 9.

**TABLE 9**

| **Solubility and precipitation assessment** | | | | | | | |
|---|---|---|---|---|---|---|---|
| **Water type** | **pH** | **Administered amount Ex. 8** | **Administered amount Ex. 9** | **Solubility Ex. 8** | **Precipitation Ex. 9** | **Solubility Ex. 8** | **Precipitation Ex. 9** |
| Non-hard | 7.5 | 0.1% | 0.2% | + | +++ | +++ | + |
| Hard | 8.5 | 0.1% | 0.2% | + | +++ | +++ | + |
| Very hard | 8.7 | 0.1% | 0.2% | + | +++ | +++ | + |

| | | | | | | | |
|---|---|---|---|---|---|---|---|
| +++ high precipitation; ++ medium precipitation; + low precipitation | | | | | | | |

According to the observed results, the composition prepared according to Example 9 (2.5% Trimethoprim) had a higher solubility in the three types of water than that of the composition in Example 8.

No problem was predicted when the compositions were administered in hard-water as they were prepared with a basic or alkaline pH, since water also has alkaline pH, therefore, no active ingredient ionization, nor formulation components ionization will be expected to occur, thus avoiding any affectation of the water supply piping system. The component of the composition prone to sedimentation is trimethoprim.

### EXAMPLE 10

### Solubility with variable doses and varying the hard-water concentration

A suspension according to the present invention was prepared having 2.5% toltrazuril and 0.5% trimethoprim, resulting a pH of 10.7, which was compared against the commercial product Baycox® (Bayer de Mexico) having 2.5% toltrazuril and a pH of 9.3. Both were tested at different doses in water having different hardness concentration measured as parts per million (ppm) of calcium carbonate, with the purpose of verifying the solubility or precipitation of both compounds once used in cattle facilities. The obtained results are shown in Table 10.

**TABLE 10**

| **Solubility of compounds having 2.5% Toltrazuril** | | | | |
|---|---|---|---|---|
| **Water hardness:** | **Product:** | **Dose: liters of Product/liters of water** | | |
| | | **1/100** | **1/250** | **1/500** |
| 100 ppm CaCO₃ in 1 liter of water | Ex. 10 suspension | Soluble | Soluble | Soluble |
| | Baycox® | Precipitates | Precipitates | Precipitates |
| 500 ppm CaCO₃ in 1 liter of water | Ex. 10 suspension | Soluble | Soluble | Soluble |
| | Baycox® | Precipitates | Precipitates | Precipitates |
| 1000 ppm CaCO₃ in 1 liter of water | Ex. 10 suspension | Soluble | Soluble | Soluble |
| | Baycox® | Precipitates | Precipitates | Precipitates |
| 1500 ppm CaCO₃ in 1 liter of water | Suspension | Soluble | Soluble | Soluble |
| | Baycox® | Precipitates | Precipitates | Precipitates |

Although in the above description some preferred embodiments of the present invention have been shown and described, it should be pointed out that several modifications thereto are possible, e.g., the pharmaceutical vehicle or the administration route of the veterinary composition. The present invention may take various forms, with the proviso that the same components are kept.

## Claims

1. A coccidicide composition of the type comprising toltrazuril: 1-methyl-3-[3-methyl-4-[4-(trifluoromethyl)tio) fenoxi]phenyl]-1,3,5-triazine(1H,3H,5H)-2,4,6-trione); trimethoprim: 5-[(3,4,5-trimethoxyphenyl)methyl]pirimidyn-2,4-diamine; and, a pharmaceutically acceptable carrier; having a basic pH, **characterized in that** the composition is an oral suspension comprising from 20 to 30 mg of toltrazuril per ml of suspension; and from 20 to 150 mg of trimethoprim per ml of suspension.

2. A coccidicide composition according to claim 1, further **characterized in that** the pH composition is from 8.5 to 12.5.

3. A coccidicide composition according to claim 1, further **characterized in that** the suspension is directly applied into the oral cavity.

4. A veterinary coccidicide composition according to claim 1, further **characterized in that** the suspension is diluted with water from a higher or equal ratio of 1:1000 (suspension/water)

5. The use of a composition as claimed in claim 1, for manufacturing a medicament useful in the treatment of coccidiosis in birds or mammals.

6. The use as claimed in claim 5, wherein the birds are poultry selected from the group comprising chicken, turkeys, ducks and gooses.

7. The use as claimed in claim 6, wherein the poultry are chicken.

## Patentansprüche

1. Kokzidiostatika-Zusammensetzung des Typs umfassend Toltrazuril: 1-Methyl-3-[3-methyl-4-[4-(trifluormethyl)thio)phenoxy]phenyl]-1,3,5-triazin(1H,3H,5H)-2,4,6-trion); Trimethoprim: 5-[(3,4,5-Trimethoxyphenyl)methyl]pirimidyn-2,4-diamin; und einen pharmazeutisch verträglichen Träger; mit einem basischen pH, **dadurch gekennzeichnet, dass** die Zusammensetzung eine orale Suspension ist, umfassend von 20 bis 30 mg Toltrazuril pro ml der Suspension; und von 20 bis 150 mg Trimethoprim pro ml der Suspension.

2. Kokzidiostatika-Zusammensetzung nach Anspruch 1, des Weiteren **dadurch gekennzeichnet, dass** der pH der Zusammensetzung von 8,5 bis 12,5 liegt.

3. Kokzidiostatika-Zusammensetzung nach Anspruch 1, des Weiteren **dadurch gekennzeichnet, dass** die Suspension direkt in die Mundhöhle verabreicht wird.

4. Veterinäre Kokzidiostatika-Zusammensetzung gemäß Anspruch 1, des Weiteren **dadurch gekennzeichnet, dass** die Suspension mit Wasser in einem höheren oder gleichen Verhältnis von 1:1000 (Suspension/Wasser) gelöst ist.

5. Verwendung einer Zusammensetzung wie in Anspruch 1 beansprucht, zum Herstellen eines Medikaments, geeignet in der Behandlung von Kokzidiose in Vögeln oder Säugern.

6. Verwendung wie in Anspruch 5 beansprucht, wobei die Vögel Geflügel sind, ausgewählt aus der Gruppe umfassend Hühner, Truthähne, Enten und Gänse.

7. Verwendung wie in Anspruch 6 beansprucht, wobei das Geflügel Hühner sind.

## Revendications

1. Composition coccidicide du type comprenant du toltrazuril : 1-méthyl-3-[3-méthyl-4-[4-(trifluorométhyl)thio)phénoxy]phényl]-1,3,5-triazine(1H,3H,5H)-2,4,6-trione) ; du triméthoprime : 5-[(3,4,5-triméthoxyphényl)méthyl]pirimidyn-2,4-diamine ; et un support pharmaceutiquement acceptable ; ayant un pH basique, **caractérisée en ce que** la composition est une suspension orale comprenant de 20 à 30 mg de toltrazuril par ml de suspension ; et de 20 à 150 mg de triméthoprime par ml de suspension.

2. Composition coccidicide selon la revendication 1, **caractérisée en outre en ce que** le pH de la composition est de 8,5 à 12,5.

3. Composition coccidicide selon la revendication 1, **caractérisée en outre en ce que** la suspension est directement appliquée dans la cavité buccale.

4. Composition coccidicide vétérinaire selon la revendication 1, **caractérisée en outre en ce que** la suspension est diluée avec de l'eau à partir d'un rapport supérieur ou égal à 1:1 000 (suspension/eau).

5. Utilisation d'une composition selon la revendication 1, pour la fabrication d'un médicament utile dans le traitement d'une coccidiose chez des oiseaux ou des mammifères.

6. Utilisation selon la revendication 5, dans laquelle les oiseaux sont des volailles choisies parmi le groupe comprenant les poulets, les dindes, les canards et les oies.

7. Utilisation selon la revendication 6, dans laquelle les volailles sont les poulets.
